# EUROPÄISCHE PATENTSCHRIFT

(11) **EP 3 804 098 B1**
(45) Veröffentlichungstag und Bekanntmachung des Hinweises auf die Patenterteilung: **27.07.2022**
(21) Anmeldenummer: 19728394.8
(22) Anmeldetag: 30.05.2019
(51) Int. Cl.: H02K 5/22, H02K 5/12, A61M 60/205, A61M 60/135

(54) **ELEKTRONIKMODUL UND ANORDNUNG FÜR EIN HERZUNTERSTÜTZUNGSSYSTEM SOWIE VERFAHREN ZUM HERSTELLEN EINES HERZUNTERSTÜTZUNGSSYSTEMS**
ELECTRONICS MODULE AND ARRANGEMENT FOR A VENTRICULAR ASSIST DEVICE, AND METHOD FOR PRODUCING A VENTRICULAR ASSIST DEVICE
MODULE ÉLECTRONIQUE ET AGENCEMENT POUR SYSTÈME D'ASSISTANCE CARDIAQUE ET PROCÉDÉ DE FABRICATION D'UN SYSTÈME D'ASSISTANCE CARDIAQUE

(30) Priorität: 30.05.2018 DE 102018208549
(43) Veröffentlichungstag der Anmeldung: 14.04.2021
(73) Patentinhaber: Kardion GmbH, 70376 Stuttgart (DE)
(72) Erfinder: STOTZ, Ingo, 71254 Ditzingen (DE); KASSEL, Julian, 75181 Pforzheim (DE); SCHUELKE, Armin, 71134 Aidlingen (DE); HENNECK, Stefan, 71229 Leonberg (DE); MINZENMAY, David, 70569 Stuttgart (DE); SCHLEBUSCH, Thomas, Alexander, 71272 Renningen (DE)
(74) Vertreter: Gauss, Nikolai
(86) Internationale Anmeldenummer: PCT/EP2019/064155
(87) Internationale Veröffentlichungsnummer: WO 2019/229221

(56) Entgegenhaltungen:
- WO-A1-99/49912
- WO-A1-2017/205909
- DE-A1-102008 060 357
- DE-C1- 19 956 380
- GB-A- 2 345 387

## Beschreibung

Die Erfindung betrifft ein Elektronikmodul und eine Anordnung für ein Herzunterstützungssystem und Verfahren zum Herstellen eines Herzunterstützungssystem.

Größere Herzunterstützungssysteme wie implantierte linksventrikuläre Unterstützungssysteme können Sensoren aufweisen, deren Elektronik beispielsweise auf Leiterplatten aufgebaut und in entsprechend große Kavitäten des Systems integriert sein kann. Kleinere, vollimplantierte Systeme, auch perkutane Unterstützungssysteme genannt, haben hohe Anforderungen an die Baugröße, sodass hier auf die Integration zusätzlicher Elektronik in der Regel verzichtet wird. Relevanter Stand der Technik ist in WO 2017/205909 A1 und GB 2 345 387 A offenbart.

Aufgabe der Erfindung ist es, ein Elektronikmodul und eine Anordnung für ein Herzunterstützungssystem sowie ein Herstellungsverfahren für ein Herzunterstützungssystem bereitzustellen, das die Integration von elektronischen Baugruppen und Sensoren in das Herzunterstützungssystem, insbesondere in einem perkutanen linkventrikulären Herzunterstützungssystem auf kleinem Baumraum ermöglicht.

Diese Aufgabe wird durch ein Herzunterstützungssystem mit den in Anspruch 1 angegebenen Merkmalen gelöst. Vorteilhafte Ausführungsformen der Erfindung sind in den abhängigen Ansprüchen angegeben. Der Erfindung liegt der Gedanke zugrunde, dass durch eine geeignet geformte Pumpen-Gehäusekomponente Sensoren und weitere elektronische Komponenten wie Mikrocontroller oder Leiterstrukturen in ein perkutanes linksventrikuläres Herzunterstützungssystem integriert werden können. Dazu wird die Pumpen-Gehäusekomponente aus mindestens zwei Gehäuseteilen realisiert, sodass die Elektronik in dem ersten Gehäuseteil aufgebaut werden kann, der Motor in dem zweiten Gehäuseteil aufgebaut werden kann und die Gehäuseteile anschließend zu einem Gesamtmotor zusammengefügt werden können. Die Elektronik kann dabei im Inneren der Pumpen-Gehäusekomponente oder in Vertiefungen auf deren Außenseite platziert sein. Letzteres ist vor allem für Sensoren sinnvoll. Beispielsweise kann die Pumpen-Gehäusekomponente zumindest abschnittsweise aus Keramik mit integrierten Leiterstrukturen gefertigt sein. Eine solche Keramikkomponente kann etwa als Spritzgussteil, in einem additiven Schichtaufbau, etwa durch 3D-Druck, oder durch Laminieren zweidimensionaler Keramiksubstrate aufgebaut werden.

Der hier vorgestellte Ansatz ermöglicht somit das hermetische Verschließen des Motors durch ein sogenanntes Backend, das Herstellen einer hermetisch abgedichteten elektrischen Kontaktierung vom Motorinneren nach außen, die Zusammenführung elektrischer Leiter aus dem Inneren des Motors mit einem auf der Außenseite des Motors geführten Sensorkabel oder einem Anschlusskabel, die Integration von Elektronik in ein perkutanes Herzunterstützungssystem als integraler Bestandteil der Gehäusekomponenten sowie die Integration von Sensorik am proximalen Ende des Herzunterstützungssystems, beispielsweise eines Blutdruck-, Beschleunigungs-, Vibrations-, Körperschall-, Temperatur-, Hall-, Ultraschall-, Oberflächenwellen-, optischen oder biochemischen Sensors oder eines Mikrofons.

Besonders vorteilhaft ist die mögliche Integration eines Mikrocontrollers als Verarbeitungseinheit in das proximale Ende zur Aggregation von Sensordaten, Speicherung von Kalibrierdaten, Identifikation über eine Seriennummer oder zur Realisierung einer Transceiver-Funktion.

Das Backend kann beispielsweise über eine 3D-MID-Keramikstruktur elektrisch funktionalisiert werden.

Durch optionale Kontaktflächen im Inneren können die Litzen der Motorwindungen direkt an die Keramik kontaktiert werden, wodurch eine zusätzliche Umverdrahtung entfallen kann.

Insbesondere ermöglicht der hier vorgestellte Ansatz eine Integration von Elektronik im Inneren einer hermetisch dichten Umgebung, sodass nur zwangsläufig zu exponierende Sensoren im nicht hermetischen Bereich Kontakt zu Blut bekommen, was Biokompatibilität und Zuverlässigkeit steigert.

Beispielsweise kann auf diese Weise ein Sensor-Hub in Form eines Mikrocontrollers zur Vorverarbeitung von Sensordaten, Übersetzung eines Kommunikationsprotokolls, Redundanz- und Fehlerabsicherung einer Übertragungsstrecke oder Identifikation einer Pumpe integriert werden.

Bei der Verwendung von Keramik können aufgrund der Isolationseigenschaften von Keramik Leiterbahnen und elektronische Komponenten ohne zusätzliche Isolationsschichten direkt auf ein Ausgangmaterial aufgebracht werden. Auch an den Kontaktierungsstellen, etwa über Pins zum Anschluss von Sensoren oder Hybridkabeln, können keine Kurzschlüsse auftreten. Zudem ermöglichen die thermischen Isolationseigenschaften von Keramik eine Entkopplung von Messungen einer Umgebungstemperatur von der Motorwärme.

Es wird ein Elektronikmodul für ein Herzunterstützungssystem vorgestellt, wobei das Herzunterstützungssystem ein Motorgehäuse zum Aufnehmen eines Pumpenmotors aufweist, wobei das Elektronikmodul folgende Merkmale aufweist:
einen Elektronikabschnitt zum Aufnehmen zumindest einer elektronischen Komponente und/oder zumindest eines elektrisch leitfähigen Kontaktierungselementes; und
einem Kopplungsabschnitt, der als Fügestelle zwischen Motorgehäuse und Elektronikabschnitt oder als separate zu fügende Komponente ausgeführt ist, wobei das Motorgehäuse und der Elektronikabschnitt über den Kopplungsabschnitt zu einem fluiddichten Modulgehäuse zur Anordnung in einem Blutgefäß kombiniert oder kombinierbar sind.

Unter einem Elektronikmodul kann eine Einheit verstanden werden, welche zur elektrischen Kontaktierung und/oder Aufnahme von Schaltungs-, Steuerungs- oder Sensortechnik ausgebildet ist. Unter einem Herzunterstützungssystem, auch Kunstherz oder VAD (ventricular assist device) genannt, kann eine Pumpeinrichtung zur Steigerung der Pumpleistung eines Herzens verstanden werden. Das Herzunterstützungssystem kann beispielsweise mittels Katheter in eine Herzkammer oder die Aorta einführbar sein. Insbesondere kann es sich bei dem Herzunterstützungssystem um ein, linksventrikuläres Unterstützungssystem handeln. Bei dem Modulgehäuse kann es sich um eine Gehäusekomponente des Herzunterstützungssystems handeln. Unter einem Kopplungsabschnitt kann ein erster Gehäuseteil des Modulgehäuses verstanden werden. Unter einem Elektronikabschnitt kann ein zweiter Gehäuseteil des Modulgehäuses verstanden werden. Das Modulgehäuse ist mit dem Motorgehäuse zu einer hermetisch abgedichteten Gehäuseeinheit verbunden, beispielsweise mittels Verschweißen. Der Kopplungs- und der Elektronikabschnitt können beispielsweise durch Stoffschluss oder unter Verwendung eines zusätzlichen Dichtelements fluiddicht miteinander verbunden sein.

Unter einer elektronischen Komponente kann beispielsweise ein Mikrocontroller, ein Sensorelement oder ein sonstiges elektronisches Bauelement, insbesondere auf Halbleiterbasis, verstanden werden. Unter einem Kontaktierungselement kann beispielsweise eine Leiterbahn, eine Leiterbahnstruktur, ein Stift, beispielsweise zur Durchkontaktierung, oder ein Pad verstanden werden.

Der Elektronikabschnitt kann beispielsweise größtenteils aus einem elektrisch isolierenden Material realisiert sein. Insbesondere kann das Kontaktierungselement dabei in das elektrisch isolierende Material zumindest abschnittsweise eingebettet sein. Unter einem Blutgefäß kann beispielsweise eine Arterie, Vene oder eine Herzkammer verstanden werden. Das Elektronikmodul kann auch als Backend des Herzunterstützungssystems bezeichnet werden und an einem proximalen Ende des Herzunterstützungssystems angeordnet sein.

Gemäß einer Ausführungsform kann der Kopplungsabschnitt als Titanteil oder Titanelement und/oder Sinterteil oder Sinterelement realisiert sein. Zusätzlich oder alternativ kann der Elektronikabschnitt als Keramikteil oder Keramikelement und/oder Schichtverbund aus zumindest zwei Schichten realisiert sein. Dadurch wird zum einen ein einfaches Kombinieren des Modulgehäuses mit dem Motorgehäuse, beispielsweise mittels Verschweißen, zum anderen eine einfache und platzsparende Integration der elektronischen Komponente oder des Kontaktierungselementes in den Elektronikabschnitt ermöglicht. Auch kann dadurch die Materialverträglichkeit oder Biokompatibilität des Elektronikmoduls verbessert werden.

Beispielsweise können die Schichten dabei in Längsrichtung des Elektronikabschnitts aufeinandergestapelt sein. Dadurch kann der Elektronikabschnitt besonders effizient gefertigt werden, etwa in einem additiven Fertigungsverfahren wie 3D-Druck.

Gemäß einer weiteren Ausführungsform kann es sich bei dem Kopplungsabschnitt um eine Oberflächenschicht oder einen Teilabschnitt des Elektronikabschnitts handeln, die vorteilhafte Fügeeigenschaften an das Motorgehäuse herstellt.

Gemäß einer weiteren Ausführungsform kann das Elektronikmodul direkt, beispielsweise durch Kleben, an das Motorgehäuse verbindbar oder verbunden sein.

Gemäß einer weiteren Ausführungsform kann der Kopplungsabschnitt mit dem Motorgehäuse verschweißt oder verschweißbar sein. Dadurch kann eine besonders zuverlässige hermetische Abdichtung des Modulgehäuses gegenüber dem Motorgehäuse erreicht werden und auf etablierte Standard-Fertigungsprozesse zurückgegriffen werden.

Der Kopplungsabschnitt kann ringförmig ausgestaltet sein. Dadurch lässt sich der Kopplungsabschnitt besonders einfach fertigen.

Gemäß einer weiteren Ausführungsform kann der Kopplungsabschnitt und/oder der Elektronikabschnitt zylinderförmig ausgestaltet sein. Dadurch kann das Elektronikmodul besonders vorteilhaft in Bezug auf eine geringe Thrombosegefahr realisiert werden.

Das Elektronikmodul kann je nach Ausführungsform die elektronische Komponente und/oder das Kontaktierungselement aufweisen. Dabei kann das Kontaktierungselement ausgebildet sein, um eine elektrisch leitfähige Kontaktierung des Elektronikmoduls über eine Außenseite des Elektronikabschnitts zu ermöglichen. Zusätzlich oder alternativ kann das Kontaktierungselement als Stift, Pad oder Leiterbahnstruktur oder eine Kombination aus zumindest zwei der genannten Ausführungsformen ausgebildet sein. Unter einem Pad kann beispielsweise eine Kontaktfläche und/oder Erhebung verstanden werden, die eine Kontaktierung nicht nur über eine punktförmige Kontaktstelle, sondern zur besseren elektrischen Kontaktierung über eine flächige Kontaktstelle ermöglicht. Zusätzlich oder alternativ kann das Kontaktierungselement zumindest abschnittsweise in ein Material des Elektronikabschnitts eingebettet sein. Dadurch können verschiedene elektrische oder elektronische Komponenten mit verhältnismäßig geringem Fertigungsaufwand in das Herzunterstützungssystem integriert werden.

Des Weiteren weist der Elektronikabschnitt eine Ausnehmung zum Aufnehmen zumindest eines Abschnitts des Pumpenmotors auf. Unter einer Ausnehmung kann im Allgemeinen eine Tasche verstanden werden. Die Ausnehmung kann beispielsweise ausgeformt sein, um den Pumpenmotor passgenau aufzunehmen. Durch diese Ausführungsform kann die Bauform des Elektronikmoduls in Längsrichtung besonders kompakt gehalten werden.

Gemäß einer Ausführungsform reicht das Kontaktierungselement in die Ausnehmung hinein, um eine elektrische Kontaktierung des von der Ausnehmung aufgenommenen Pumpenmotors zu ermöglichen. Dabei kann sich der Kopplungsabschnitt um die Ausnehmung herum, beispielsweise ringförmig, erstrecken, sodass ein Hindurchführen des Pumpenmotors durch den Kopplungsabschnitt in die Ausnehmung möglich ist. Durch diese Ausführungsform wird eine besonders einfache elektrische Kontaktierung des Pumpenmotors ohne zusätzliche Kontaktierungselemente ermöglicht.

Von Vorteil ist auch, wenn der Elektronikabschnitt einen Sensorabschnitt zum Aufnehmen zumindest eines Sensorelements und/oder einen Anschlussabschnitt zum Anschließen eines Kabels an das Elektronikmodul aufweist. Dabei kann das Kontaktierungselement an dem Sensorabschnitt und/oder dem Anschlussabschnitt angeordnet oder anordenbar sein. Bei dem Sensorabschnitt kann es sich beispielsweise um einen abgeflachten Bereich einer Mantelfläche des Elektronikabschnitts handeln. Unter einem Anschlussabschnitt kann beispielsweise eine Stirnseite des Elektronikmoduls verstanden werden. Beispielsweise kann der Anschlussabschnitt als Anschlussfeld mit einer Mehrzahl von Kontaktierungselementen in Form von Anschlusspins oder Kontaktmulden zum Kontaktieren eines Kabels oder eines Steckverbinders realisiert sein. Dadurch werden eine einfache Anbringung von Sensorelementen und eine einfache elektrische Kontaktierung des Elektronikmoduls ermöglicht.

Gemäß einer Ausführungsform kann der Anschlussabschnitt an einer dem Kopplungsabschnitt abgewandten Stirnseite des Elektronikabschnitts angeordnet sein. Zusätzlich oder alternativ kann der Elektronikabschnitt mit einer Schutzkappe, einem Zugentlastungselement, einem Knickschutzelement oder einer Kombination aus zumindest zwei der vorgenannten Elemente gekoppelt oder koppelbar sein. Dadurch können Beschädigungen des Elektronikmoduls vermieden werden. Zudem wird dadurch ein einfaches Einführen des Elektronikmoduls in das Blutgefäß ermöglicht.

Gemäß einer weiteren Ausführungsform kann der Anschlussabschnitt mit einer Mehrzahl von Kontaktmulden zur elektrischen Kontaktierung des Kabels ausgeformt sein. Unter einer Kontaktmulde kann eine beispielsweise halbkreisförmige Vertiefung verstanden werden, die mit einem elektrisch leitfähigen Material ausgekleidet sein kann. Die Kontaktmulden können beispielsweise um eine Mittelachse des Elektronikabschnitts herum angeordnet sein. Dadurch wird eine steckerlose elektrische Kontaktierung des Elektronikmoduls ermöglicht, beispielsweise durch Verlöten einzelner Adern des Kabels mit dem elektrisch leitfähigen Material in den Kontaktmulden.

Der hier vorgestellte Ansatz schafft ferner ein Herzunterstützungssystem mit folgenden Merkmalen:
einem Motorgehäuse zum Aufnehmen eines Pumpenmotors; und
einem Elektronikmodul mit einem Elektronikabschnitt und einem Kopplungsabschnitt zum Aufnehmen zumindest einer elektronischen Komponente und/oder zumindest eines elektrisch leitfähigen Kontaktierungselementes, wobei der Elektronikabschnitt und der Kopplungsabschnitt oder das Motorgehäuse miteinander zu einem fluiddichten Modulgehäuse, zur Anordnung in einem Blutgefäß kombiniert sind, wobei der Elektronikabschnitt über den Kopplungsabschnitt fluiddicht mit dem Motorgehäuse gekoppelt ist.

Schließlich schafft der vorgestellte Ansatz ein Verfahren zum Herstellen eines Elektronikmoduls für ein Herzunterstützungssystem, das nicht von den Ansprüchen umfasst ist, wobei das Herzunterstützungssystem ein Motorgehäuse zum Aufnehmen eines Pumpenmotors aufweist, wobei das Verfahren folgende Schritte umfasst:
Bilden eines Elektronikabschnitts zum Aufnehmen zumindest einer elektronischen Komponente und/oder zumindest eines elektrisch leitfähigen Kontaktierungselementes an einem Kopplungsabschnitt, wobei der Kopplungsabschnitt ausgeformt ist, um das Elektronikmodul fluiddicht mit dem Motorgehäuse zu koppeln.

Ferner kann in einer besonders günstigen Ausführungsform ein optionaler Schritt des Kombinierens der Elektronikabschnitt mit einem als separaten Element ausgeführten Kopplungsabschnitt zu einem fluiddichten Modulgehäuse zur Anordnung in einem Blutgefäß kombiniert werden, um das Elektronikmodul fluiddicht mit dem Motorgehäuse zu koppeln.

Das Ausformen des optionalen separaten Elementes als Kopplungsabschnitts kann beispielsweise durch Sintern erfolgen. Insbesondere kann der Kopplungsabschnitt im Schritt des Kombinierens direkt an den Elektronikabschnitt angesintert werden. Das Bilden des Elektronikabschnitts kann beispielsweise in einem additiven Fertigungsverfahren, insbesondere durch Aufeinanderstapein von Keramikschichten, erfolgen. Alternativ kann das Bilden des Elektronikabschnitts in einem Spritzgießverfahren oder einem spanenden Fertigungsverfahren, etwa durch Fräsen oder Drehen, erfolgen. Das Kontaktierungselement kann im Schritt des Bildens direkt in den Elektronikabschnitt eingebettet werden, beispielsweise in Form eines Stifts zur Durchkontaktierung einzelner Schichten des Elektronikabschnitts. Zusätzlich oder alternativ kann das Kontaktierungselement auf eine Oberfläche des Elektronikabschnitts oder einzelner Schichten davon aufgebracht werden.

Vorteilhafte Ausführungsbeispiele der Erfindung sind anhand der Zeichnungen in der nachfolgenden Beschreibung näher erläutert.

Es zeigen:
- Fig. 1: eine schematische Darstellung eines Herzunterstützungssystems gemäß einem Ausführungsbeispiel;
- Fig. 2A: eine schematische Darstellung eines Elektronikmoduls aus Fig. 1;
- Fig. 2B: schematische Darstellung eines Elektronikmoduls zur direkten Verbindung mit dem Motorgehäuse, beispielsweise durch Kleben;
- Fig. 3: eine schematische Darstellung eines Schichtaufbaus eines Elektronikabschnitts gemäß einem Ausführungsbeispiel;
- Fig. 4: eine schematische Darstellung eines Elektronikmoduls gemäß einem Ausführungsbeispiel;
- Fig. 5: eine schematische Darstellung des Elektronikmoduls aus Fig. 4;
- Fig. 6: eine schematische Darstellung eines Elektronikmoduls gemäß einem Ausführungsbeispiel;
- Fig. 7: ein Ablaufdiagramm eines Verfahren zum Herstellen eines Elektronikmoduls gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

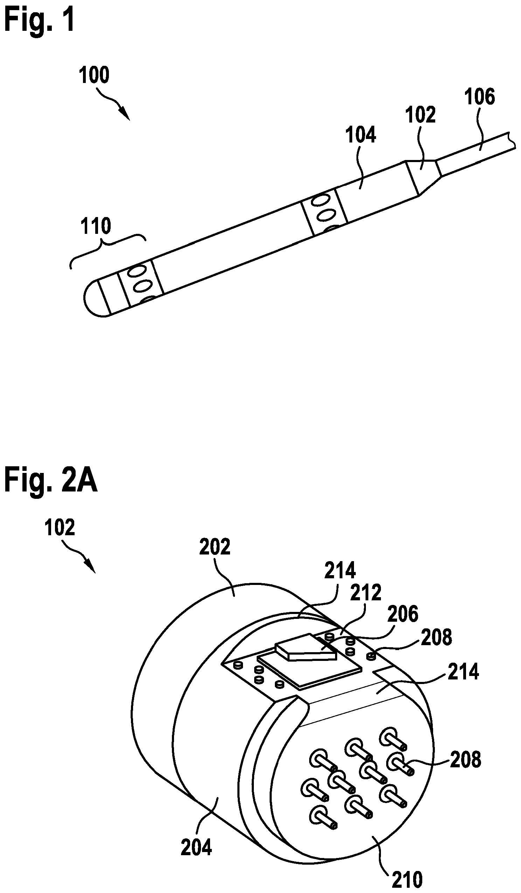

Fig. 1 zeigt eine schematische Darstellung eines Herzunterstützungssystems 100 gemäß einem Ausführungsbeispiel. Das Herzunterstützungssystem 100, hier beispielhaft ein linksventrikuläres Herzunterstützungssystem zur perkutanen Implantierung in eine linke Herzkammer, weist ein Elektronikmodul 102 auf, das mit einem Motorgehäuse 104 zum Aufnehmen eines Pumpenmotors fluiddicht verbunden ist. Das Elektronikmodul 102 repräsentiert dabei ein proximales Ende des Herzunterstützungssystems 100 und bildet einen Übergang zwischen dem Motorgehäuse 104 und einem Anschlusskabel 106 zum Anschließen des Herzunterstützungssystems 100 an eine externe Energiequelle oder eine externe Auswerte- oder Steuereinrichtung.

Das Herzunterstützungssystem 100 weist einen zylinderförmigen, länglichen Aufbau mit im Wesentlichen konstantem Außendurchmesser und abgerundeten, sich verjüngenden Enden zur einfachen Platzierung mittels Katheter in einem Blutgefäß, etwa der linken Herzkammer oder der Aorta, auf. Beispielhaft weist das Herzunterstützungssystem 100 eine Spitze in Form einer Sensorbaugruppe 110 auf, etwa zur Druckmessung.

Fig. 2A zeigt eine schematische Darstellung des Elektronikmoduls 102 aus Fig. 1. Das Elektronikmodul 102 umfasst ein zylinderförmiges Modulgehäuse, das aus einem (optionalen) Kopplungsabschnitt 202 und einem Elektronikabschnitt 204 zusammengesetzt ist. Der Kopplungsabschnitt 202 dient zum fluiddichten Koppeln des Elektronikmoduls 102 mit dem Motorgehäuse, beispielsweise durch Verschweißen. Für Verklebungen kann auch eine direkte Fügung stattfinden. Gemäß einem günstigen Ausführungsbeispiel ist der Kopplungsabschnitt 202 als (Metall-)Ring, insbesondere als Titanring realisiert. Der Elektronikabschnitt 204 dient zum Aufnehmen einer oder mehrerer elektronischer Komponenten 206. Zusätzlich weist der Elektronikabschnitt 204 gemäß diesem Ausführungsbeispiel eine Mehrzahl elektrisch leitfähiger Kontaktierungselemente 208 auf, die zur elektrischen Kontaktierung der elektronischen Komponente 206 oder des Pumpenmotors oder zum Anschließen eines Kabels an den Elektronikabschnitt 204 ausgebildet sind.

Die beiden Abschnitte 202, 204 (soweit der Kopplungsabschnitt 202 als separates Element ausgeformt ist) sind beispielsweise durch Stoffschluss fluiddicht miteinander verbunden. Alternativ kann auch das Motorgehäuse direkt auf einen als Kopplungsabschnitt 202 wirkenden Oberflächenteil des Elektronikabschnitts 204 aufgebracht und fluiddicht mit demselbsen verschlossen werden.

Ein Teil der Kontaktierungselemente 208 ist gemäß Fig. 2A an einem stirnseitigen, dem Kopplungsabschnitt 202 und/oder der Motorgehäuseseite abgewandten Anschlussabschnitt 210 des Elektronikabschnitts 204 angeordnet, wobei aus dem Anschlussabschnitt 210 herausragende Enden der Kontaktierungselemente 208 als Anschlussstifte zum Anschluss eines Anschlusskabels ausgebildet sind. Die Kontaktierung des Anschlusskabels erfolgt entsprechend über Kontakthülsen oder eine direkte Verbindung. Der Anschlussabschnitt 210 kann auch als Anschlussfeld bezeichnet werden. Die elektronische Komponente 206, hier ein Sensorelement, ist auf einem außenliegenden Sensorabschnitt 212 des Elektronikabschnitts 204 platziert. Der Sensorabschnitt 212 ist hier als Abflachung einer Mantelfläche des Elektronikabschnitts 204 realisiert und bildet somit eine Plattform für das Sensorelement 206. Auch sind im Sensorabschnitt 212 mehrere der Kontaktierungselemente 208 zur Kontaktierung in ein hermetisches Innere des Elektronikabschnitts 204 angeordnet.

Das Elektronikmodul 102, das auch als Backend des Herzunterstützungssystems bezeichnet werden kann, ist beispielsweise mit einem Keramikteil als Elektronikabschnitt 204 und einem angesinterten Titanring als Kopplungsabschnitt 202 realisiert. Der zweiteilige Aufbau ist vorteilhaft, da dadurch anschließende Produktionsschritte, wie etwa ein hermetisches Verschweißen des Elektronikmoduls 102 mit dem Motorgehäuse, vereinfacht werden. Die Verwendung von Keramik als Gehäusematerial bietet vor allem den Vorteil einer einfachen Integration komplexer Leiterstrukturen. Durch die Verwendung eines Titanelements als Kopplungsabschnitt 202 wird hingegen eine hermetische Verschweißung mit dem Motorgehäuse gewährleistet.

Der Elektronikabschnitt 204 ist beispielsweise durch einen schichtweisen Aufbau nach der cofiring-Technik als funktionalisierte Keramikkomponente realisiert.

Gemäß einem Ausführungsbeispiel ist der Kopplungsabschnitt 202 formschlüssig verglast, um eine hermetisch dichte Verbindung zwischen dem Kopplungsabschnitt 202 und dem Elektronikabschnitt 204 herzustellen.

Der Elektronikabschnitt 204 ist optional mit einer oder mehreren verrundeten Stufen 214 ausgeformt, sodass beispielsweise ein Dünnschichtsubstrat zur Anbindung weiterer Sensoren aus der in Fig. 1 gezeigten Sensorbaugruppe ohne Beschädigung zum Anschlussabschnitt 210 geführt werden kann. Der Anschlussabschnitt 210 dient beispielsweise zur Anbindung hermetischer Durchführungspins an das Anschlusskabel.

Optional ist eine weitere Stufe 216 zumindest abschnittsweise um den Anschlussabschnitt 210 herum ausgeformt, um die Anbindung einer Schutzkappe, Zugentlastung oder Knickschutztülle zu ermöglichen.

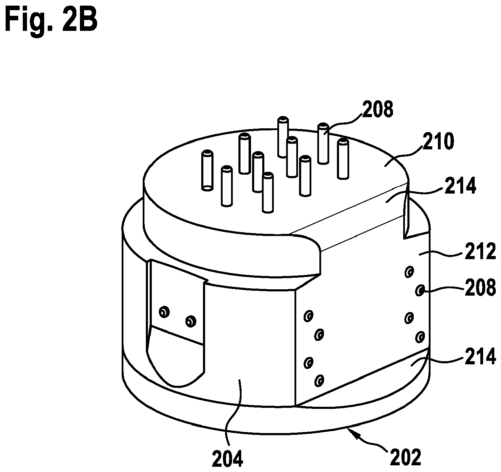

Fig. 2B zeigt eine schematische Darstellung eines Elektronikmoduls 102 gemäß einem weiteren Ausführungsbeispiel zur direkten Verbindung mit dem Motorgehäuse, beispielsweise durch Kleben. Hierbei ist erkennbar, dass das Elektronikmodul 102 einen Kopplungsabschnitt 202 aufweist, der als Oberflächenschicht bzw. als Teilabschnitt des Elektronikabschnitts 204 ausgeformt ist, nicht jedoch als separates Element, wie es in der Fig. 2A dargestellt ist.

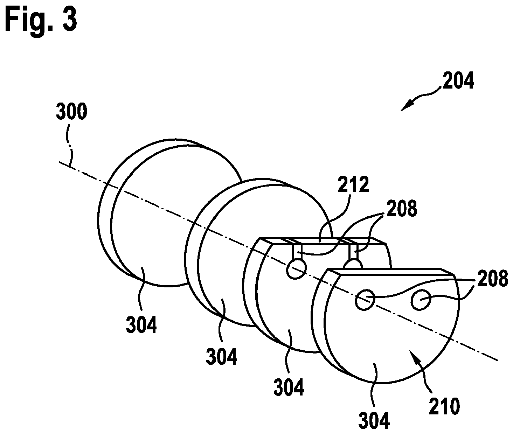

Fig. 3 zeigt eine schematische Darstellung eines Schichtaufbaus eines Elektronikabschnitts 204 gemäß einem Ausführungsbeispiel. Beispielhaft gezeigt ist ein Lagenaufbau des Elektronikabschnitts 204, wie er in einem LTCC-Verfahren (LTCC = Low Temperature Cofired Ceramics; "Niedertemperatur-Einbrand-Keramiken") realisierbar ist. Wie zu erkennen, umfasst der Schichtaufbau dabei eine Mehrzahl von entlang einer Längsachse 300 des Elektronikabschnitts 204 aufeinandergestapelter Einzelschichten 304, hier eine Mehrzahl von scheibenförmigen Keramiklagen.

Leiterstrukturen aus den Kontaktierungselementen 208 sind beispielsweise in der Ebene der Einzelschichten 304 durch Siebdruck und Vias von Einzelschicht zu Einzelschicht realisiert.

Das Elektronikmodul wird beispielsweise Schritt für Schritt durch Stapeln gestanzter und leitfähig bedruckter Grünteile aufgebaut. Dadurch ergibt sich die Randbedingung, dass weiter oben liegende Lagen nur identische oder kleinere Abmaße als tiefere Lagen haben dürfen. Anderslautende Formanforderungen sind beispielsweise durch eine entsprechende Nachbearbeitung von Sinterteilen des Elektronikmoduls durch Fräsen oder Drehen realisierbar.

Alternativ ist der Elektronikabschnitt 204 durch 3D-Druck, Spritzguss oder Fräsen als funktionalisierte Keramikkomponente hergestellt. Die elektrische Funktionalisierung erfolgt anschließend durch Siebdruck, Dispensen und verglaste Durchführungen.

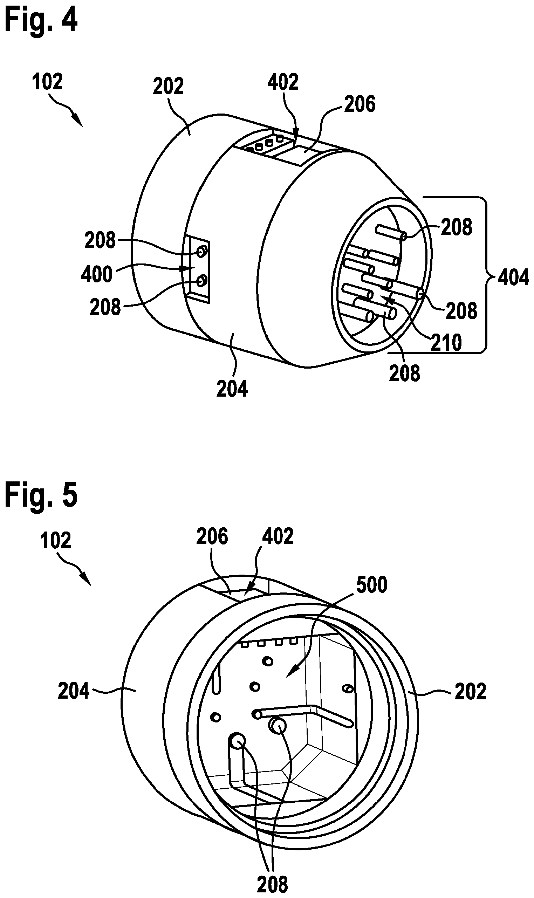

Fig. 4 zeigt eine schematische Darstellung eines Elektronikmoduls 102 gemäß einem Ausführungsbeispiel. Gezeigt ist eine Ausführungsform des Elektronikmoduls 102 als Keramik-MID-Teil. Im Unterschied zu dem vorangehend anhand der Figuren 1 bis 3 beschriebenen Elektronikmodul umfasst die Mantelfläche des Elektronikabschnitts 204 hier eine erste Kavität 400, in die beispielhaft zwei Kontaktierungselemente 208 hineinreichen, und eine zweite Kavität 402, deren Bodenfläche den Sensorabschnitt zur Anordnung der Komponente 206 bildet. In einer dritten, stirnseitigen Kavität 404 sind die Kontaktierungselemente 208 des Anschlussabschnitts 210 angeordnet, wobei eine Bodenfläche der dritten Kavität 404 den Anschlussabschnitt 210 bildet. Optional ist der Elektronikabschnitt 204 im Bereich des Anschlussabschnitts 210 konusförmig ausgestaltet.

Durch die vorgenannten Fertigungsverfahren sind in begrenztem Rahmen Formschlüsse ohne Nachbearbeitung abbildbar, sodass neben Plattformen für Sensoren auch Kavitäten an der Mantel- oder Stirnfläche des Elektronikabschnitts 204 realisierbar sind, wie sie beispielhaft in Fig. 4 gezeigt sind. Dadurch kann etwa der mechanische Schutz des außenliegenden Sensorelements 206 und des Anschlussabschnitts 210 verbessert werden.

Fig. 5 zeigt eine schematische Darstellung des Elektronikmoduls 102 aus Fig. 4. Gezeigt ist eine Innenansicht des Elektronikmoduls 102 mit exemplarischen Leiterstrukturen. Es ist zu erkennen, dass der Elektronikabschnitt 204 eine innenliegende Ausnehmung 500 aufweist, in die eine Mehrzahl der Kontaktierungselemente 208 hineinreicht. Die Ausnehmung 500 dient beispielsweise zur Aufnahme einer Anschlussseite des Pumpenmotors, um eine direkte elektrische Kontaktierung des Pumpenmotors mit den Kontaktierungselementen 208 zu ermöglichen. Dabei ist es zweckdienlich, wenn der Kopplungsabschnitt 202 als Ring realisiert ist.

Die durch die Kontaktierungselemente 208 gebildeten elektrischen Leiterstrukturen sind beispielsweise außen durch Siebdruck oder innen durch Dispensen hergestellt. In Fig. 5 ist ein entsprechendes Ausführungsbeispiel gezeigt. Beim Dispensen führt ein Roboterarm eine dünne Kanüle, durch die leitfähiges Material wie Goldpaste oder Leitkleber abgegeben wird. Durch einen Sinterprozess wird die leitfähige Paste fest mit der Keramik verbunden, um die elektrischen Leiterbahnen zu realisieren.

Das Sensorelement 206 ist gemäß einem Ausführungsbeispiel als Sensor-Hub, etwa in Form eines Mikrocontrollers, realisiert, um Kalibrier- und Identifikationsinformationen des Pumpenmotors oder von Sensoren zu erfassen. Dabei ist das Sensorelement 206 beispielsweise über einen Kommunikationsbus im Anschlusskabel über ein zentrales Steuergerät des Herzunterstützungssystems auslesbar. Dadurch kann das Steuergerät beispielsweise mit Motordaten parametriert werden.

Das Sensorelement 206 ist beispielsweise ausgebildet, um Sensordaten von Sensoren des Pumpenmotors vorzuverarbeiten, etwa zu aggregieren, zu filtern oder zu kalibrieren, oder als Transceiver ein Kommunikationsprotokoll der Sensoren in ein robusteres Kommunikationsprotokoll zu übersetzen oder künstliche Redundanz oder Prüfsummen hinzuzufügen.

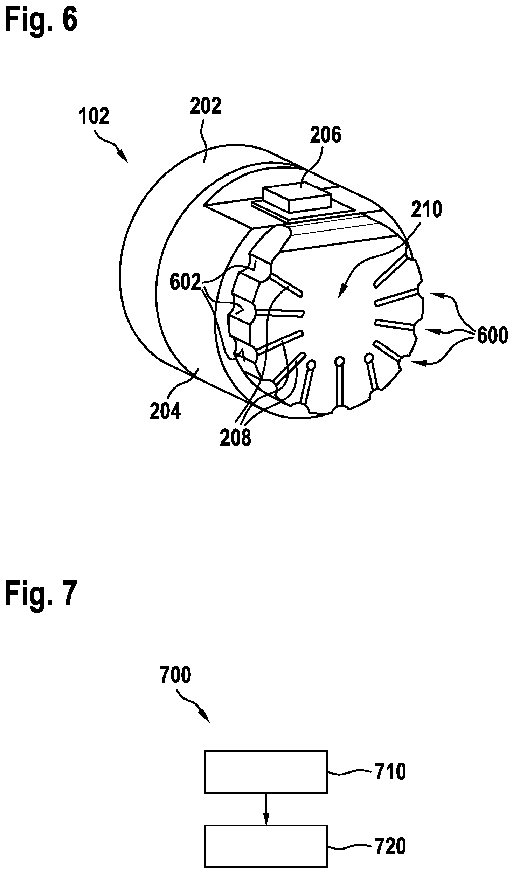

Fig. 6 zeigt eine schematische Darstellung eines Elektronikmoduls 102 gemäß einem Ausführungsbeispiel. Das Elektronikmodul 102 entspricht im Wesentlichen dem vorangehend anhand von Fig. 2 beschriebenen Elektronikmodul, mit dem Unterschied, dass der Anschlussabschnitt 210 eine Mehrzahl von bogenförmig, beispielsweise konzentrisch zu einer Umfangslinie des Elektronikabschnitts 204 angeordneten Kontaktmulden 600 zur elektrischen Kontaktierung des Anschlusskabels, etwa einzelner Adern des Anschlusskabels, aufweist. Die Kontaktierungselemente 208 weisen dabei je eine elektrisch leitfähige Kontaktierungsfläche 602 auf, die je eine der Kontaktmulden 600 auskleidet.

Die Kontaktmulden 600 dienen insbesondere zur direkten Kontaktierung des Anschlusskabels an die Keramik. Die Litzen des Anschlusskabels sind dabei direkt elektrisch an die außen platzierten Kontaktmulden 600 in der Keramik kontaktiert. Ein Dünnschichtsubstrat ist beispielsweise durch Blindpins oder Kontaktpads in der Mitte des Anschlussabschnitts 210 und eine kurze Verbindungsleitung an die Kontaktmulden 600 angebunden. Um eine Delaminierung der mechanisch belasteten Kontaktierungsflächen 602 zu verhindern, ist beispielsweise ein isolierender Keramikring zur mechanischen Stabilisierung über den Anschlussabschnitt 210 geschoben.

Fig. 7 zeigt ein Ablaufdiagramm eines Verfahrens 700 zum Herstellen eines Elektronikmoduls.

Dabei wird in einem ersten Schritt 710 der Elektronikabschnitt zum Aufnehmen zumindest einer elektronischen Komponente und/oder zumindest eines elektrisch leitfähigen Kontaktierungselementes an einem Kopplungsabschnitt gebildet, wobei der Kopplungsabschnitt ausgeformt ist, um das Elektronikmodul fluiddicht mit dem Motorgehäuse zu koppeln. In einem zweiten Schritt 720 kann der Elektronikabschnitt mit dem Kopplungsabschnitt kombiniert werden, um das hermetisch abgedichtete Modulgehäuse herzustellen.

Gemäß einem Beispiel erfolgt das Kombinieren im Schritt 720 durch Ansintern des Kopplungsabschnitts an den Elektronikabschnitt. Alternativ erfolgt das Kombinieren durch Fügen des Kopplungs- und des Elektronikabschnitts. Alternativ kann das Kopplungselement aus Metall, im Besonderen Titanfolie, ausgeführt sein und der Kombinationsschritt 720 auf dem Diffusionsschweißen des folienförmigen Kopplungselements an das Elektronikelement erfolgen. Auch kann das Kopplungselement als metallische Oberflächenbeschichtung des Elektronikelements ausgeführt sein, sodass die hermetische Fügung an das Motorgehäuse im Folgenden durch beispielsweise einen Laserlötprozess möglich wird.

Gemäß einem weiteren Beispiel wird der Elektronikabschnitt im Schritt 710 schichtweise als Keramikbauteil hergestellt.

Die Fügung des hermetischen Modulgehäuses an das Motorgehäuse kann durch Schweißen eines optionalen Kopplungselements, beispielsweise aus Titan, erfolgen. In einer anderen Ausführung kann die Fügung durch Reaktiv-Bonden des Motorgehäuses (beispielsweise aus Titan) an das Modulgehäuse (beispielsweise aus Oxidkeramik) erfolgen. Alternativ ist eine Fügung des Elektronikabschnitts an das Motorgehäuse beispielsweise durch Verklebung mit einem beispielsweise Kunstharz möglich.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Herzunterstützungssystem mit einer in eine Längsrichtung erstreckten zylinderförmigen Struktur, die durch einen Katheter in die Aorta oder in eine Herzkammer einführbar ist und die eine Spitze aufweist **dadurch gekennzeichnet, dass**
die zylinderförmige Struktur einen in einem Motorgehäuse (104) angeordneten Pumpenmotor enthält und ein Elektronikmodul (102) mit einem Elektronikabschnitt (204) umfasst, der einen Kopplungsabschnitt (202) mit einer Ausnehmung (500) zum Aufnehmen zumindest eines Abschnitts des Pumpenmotors aufweist, in der für eine elektrische Kontaktierung des Pumpenmotors ein elektrisch leitfähiges Kontaktierungselement (208) angeordnet ist das zumindest abschnittsweise in ein Material des Elektronikabschnitts eingebettet und zu einer Außenseite des Körpers des Elektronikabschnitts an einen Stift oder einen Pad oder eine Leiterbahnstruktur geführt ist, wobei der Körper des Elektronikabschnitts (204) mit dem Kopplungsabschnitt (202) und dem Motorgehäuse (104) ein fluiddichtes Modulgehäuse bilden.

2. Herzunterstützungssystem nach Anspruch 1, **dadurch gekennzeichnet, dass** der Kopplungsabschnitt einen Abschnitt aus der Gruppe Titanelement, Titanteil, Sinterelement, Keramikelement enthält.

3. Herzunterstützungssystem nach Anspruch 1 oder 2, **dadurch gekennzeichnet, dass** der Elektronikabschnitt (204) als ein Keramikelement realisiert ist.

4. Herzunterstützungssystem nach einem der Ansprüche 1 bis 3, **dadurch gekennzeichnet, dass** der Elektronikabschnitt (204) als ein Schichtverbund aus zumindest zwei Schichten (304) realisiert ist.

5. Herzunterstützungssystem nach Anspruch 4, **dadurch gekennzeichnet, dass** die Schichten (304) in Längsrichtung des Elektronikabschnitts (204) aufeinandergestapelt sind.

6. Herzunterstützungssystem nach einem der Ansprüche 1 bis 5, **dadurch gekennzeichnet, dass** das Elektronikmodul (102) mit dem Motorgehäuse (104) verklebt ist.

7. Herzunterstützungssystem nach einem der vorangegangenen Ansprüche 1 bis 6, **dadurch gekennzeichnet, dass** der Kopplungsabschnitt (202) mit dem Motorgehäuse (104) verschweißt ist.

8. Herzunterstützungssystem nach einem der Ansprüche 1 bis 7, **dadurch gekennzeichnet, dass** der Elektronikabschnitt (204) einen Sensorabschnitt (212) zum Aufnehmen zumindest eines Sensorelements (206) aufweist.

9. Herzunterstützungssystem nach einem der Ansprüche 1 bis 8, **gekennzeichnet durch** einen Anschlussabschnitt (210) zum Anschließen eines Kabels (106) an das Elektronikmodul (102), wobei das Kontaktierungselement (208) an dem Anschlussabschnitt (210) angeordnet ist.

10. Herzunterstützungssystem nach Anspruch 9, **dadurch gekennzeichnet, dass** der Anschlussabschnitt (210) mit einer Mehrzahl von Kontaktmulden (600) zur elektrischen Kontaktierung des Kabels (106) ausgeformt ist.

11. Herzunterstützungssystem nach einem der Ansprüche 9 oder 10, **dadurch gekennzeichnet, dass** der Anschlussabschnitt (210) an einer dem Kopplungsabschnitt (202) abgewandten Stirnseite des Elektronikabschnitts (204) angeordnet ist und/oder mit einer Schutzkappe und/oder einem Zugentlastungselement und/oder einem Knickschutzelement gekoppelt oder koppelbar ist.

## Claims

1. Cardiac support system comprising a cylindrical structure extending in a longitudinal direction, which can be inserted through a catheter into the aorta or into a heart chamber and which has a tip,
**characterized in that**
the cylindrical structure contains a pump motor arranged in a motor housing (104) and comprises an electronics module (102) having an electronics portion (204), which electronics portion has a coupling portion (202) that has a recess (500) for receiving at least a portion of the pump motor, in which recess an electrically conductive contacting element (208) is arranged for electrical contacting of the pump motor, which contacting element is embedded at least in part in a material of the electronics portion and is routed to an external face of the body of the electronics portion on a pin or a pad or a conductor track structure, the body of the electronics portion (204) forming a fluid-tight module housing together with the coupling portion (202) and the motor housing (104).

2. Cardiac support system according to claim 1, **characterized in that** the coupling portion contains a portion from the group consisting of a titanium element, a titanium part, a sintered element, a ceramic element.

3. Cardiac support system according to either claim 1 or claim 2, **characterized in that** the electronics portion (204) is implemented as a ceramic element.

4. Cardiac support system according to any of claims 1 to 3, **characterized in that** the electronics portion (204) is implemented as a layered composite of at least two layers (304).

5. Cardiac support system according to claim 4, **characterized in that** the layers (304) are stacked on top of one another in the longitudinal direction of the electronics portion (204).

6. Cardiac support system according to any of claims 1 to 5, **characterized in that** the electronics module (102) is bonded to the motor housing (104).

7. Cardiac support system according to any of the preceding claims 1 to 6, **characterized in that** the coupling portion (202) is welded to the motor housing (104).

8. Cardiac support system according to any of claims 1 to 7, **characterized in that** the electronics portion (204) has a sensor portion (212) for receiving at least one sensor element (206).

9. Cardiac support system according to any of claims 1 to 8, **characterized by** a connection portion (210) for connecting a cable (106) to the electronics module (102), the contacting element (208) being arranged on the connection portion (210).

10. Cardiac support system according to claim 9, **characterized in that** the connection portion (210) is formed having a plurality of contact depressions (600) for electrically contacting the cable (106).

11. Cardiac support system according to either claim 9 or claim 10, **characterized in that** the connection portion (210) is arranged on an end face of the electronics portion (204) facing away from the coupling portion (202) and/or is or can be coupled to a protective cap and/or a strain relief element and/or an anti-kink element.

## Revendications

1. Système d'assistance cardiaque comportant une structure cylindrique s'étendant dans une direction longitudinale, qui peut être insérée à travers un cathéter dans l'aorte ou dans un ventricule et qui présente une pointe
**caractérisé en ce que**
la structure cylindrique contient un moteur de pompe disposé dans un carter de moteur (104) et comprend un module électronique (102) comportant une section électronique (204), lequel présente une section de couplage (202) comportant un évidement (500) permettant de recevoir au moins une section du moteur de pompe, dans lequel module électronique un élément de contact (208) électriquement conducteur est disposé pour établir un contact électrique avec le moteur de pompe, lequel élément de contact est incorporé, au moins dans certaines régions, dans un matériau de la section électronique et est acheminé vers une face extérieure du corps de la section électronique sur une broche ou une pastille ou une structure de pistes conductrices, le corps de la section électronique (204) formant un boîtier de module étanche aux fluides avec la section de couplage (202) et le carter de moteur (104).

2. Système d'assistance cardiaque selon la revendication 1, **caractérisé en ce que** la section de couplage contient une section du groupe constitué d'un élément en titane, d'une pièce en titane, d'un élément fritté et un d'élément en céramique.

3. Système d'assistance cardiaque selon la revendication 1 ou 2, **caractérisé en ce que** la section électronique (204) est réalisée sous la forme d'un élément en céramique.

4. Système d'assistance cardiaque selon l'une des revendications 1 à 3, **caractérisé en ce que** la section électronique (204) est réalisée sous la forme d'un composite stratifié d'au moins deux couches (304).

5. Système d'assistance cardiaque selon la revendication 4, **caractérisé en ce que** les couches (304) sont empilées les unes sur les autres dans la direction longitudinale de la section électronique (204).

6. Système d'assistance cardiaque selon l'une des revendications 1 à 5, **caractérisé en ce que** le module électronique (102) est collé au carter de moteur (104).

7. Système d'assistance cardiaque selon l'une des revendications précédentes 1 à 6, **caractérisé en ce que** la section de couplage (202) est soudée au carter de moteur (104).

8. Système d'assistance cardiaque selon l'une des revendications 1 à 7, **caractérisé en ce que** la section électronique (204) présente une section de capteur (212) permettant de recevoir au moins un élément de capteur (206).

9. Système d'assistance cardiaque selon l'une des revendications 1 à 8, **caractérisé par** une section de connexion (210) permettant de connecter un câble (106) au module électronique (102), l'élément de contact (208) étant disposé sur la section de connexion (210).

10. Système d'assistance cardiaque selon la revendication 9, **caractérisé en ce que** la section de connexion (210) est formée avec une pluralité de dépressions de contact (600) permettant d'établir un contact électrique avec le câble (106).

11. Système d'assistance cardiaque selon l'une des revendications 9 ou 10, **caractérisé en ce que** la section de connexion (210) est disposée sur une face frontale de la section électronique (204) opposée à la section de couplage (202) et/ou est couplée ou peut être couplée avec un capuchon de protection et/ou un élément de décharge de traction et/ou un élément antipli.
